# EUROPÄISCHE PATENTSCHRIFT

(11) **EP 2 387 422 B1**
(45) Veröffentlichungstag und Bekanntmachung des Hinweises auf die Patenterteilung: **12.10.2016**
(21) Anmeldenummer: 10700377.4
(22) Anmeldetag: 12.01.2010
(51) Int. Cl.: A61L 2/20, A61L 2/22, A61L 2/24, A61L 101/10, A61L 101/22

(54) **VERFAHREN UND VORRICHTUNG ZUR STERILISATION EINES ODER MEHRERER GEGENSTÄNDE**
METHOD AND DEVICE FOR STERILIZING ONE OR MORE OBJECTS
PROCÉDÉ ET DISPOSITIF DE STÉRILISATION D'UN OU DE PLUSIEURS OBJETS

(30) Priorität: 14.01.2009 DE 102009004589
(43) Veröffentlichungstag der Anmeldung: 23.11.2011
(73) Patentinhaber: Fresenius Medical Care Deutschland GmbH, 61352 Bad Homburg (DE)
(72) Erfinder: DRÖSCHEL, Stefan, 66125 Saarbrücken (DE); GROSS, Arnold, 66629 Freisen (DE)
(74) Vertreter: Herrmann, Uwe
(86) Internationale Anmeldenummer: PCT/EP2010/000105
(87) Internationale Veröffentlichungsnummer: WO 2010/081672

(56) Entgegenhaltungen:
- EP-A1- 0 016 887
- WO-A1-03/039607
- WO-A1-2007/014435
- DE-A1- 19 858 344
- US-A- 4 335 071
- US-A- 4 971 761
- "Liste der vom Robert-Koch-Institut geprüften und anerkannten Desinfektionsmittel und-verfahren", BUNDESGESUNDHEITSBLATT, HEYMANN, KOELN, DE, 15. Juni 1997 (1997-06-15), Seiten 344-358, XP002182338, ISSN: 0007-5914
- Axel Kramer, Ojan Assadian: "Wallhäusers Praxis der Sterilisation, Desinfektion, Antiseptik und Konservierung", 2008, Georg Thieme Verlag, Stuttgart, New York, XP002584630, ISBN: 9783131411211 Seiten 64-65, Absatz 5.6.7.3; Seite 64 - Seite 65; Abbildung 5.12

## Beschreibung

Die vorliegende Erfindung betrifft ein Verfahren sowie eine Vorrichtung zur Sterilisation eines oder mehrerer Gegenstände.

Die heute zur Anwendung kommenden Sterilisationsverfahren, wie. z. B. die sogenannte ETO - Sterilisation, die Gamma-Bestrahlung oder auch die Dampfsterilisation sind für bestimmte Sterilisationsvorgänge ungeeignet, da sie teilweise mit hohen Temperaturen arbeiten und so gegebenenfalls den zu sterilisierenden Gegenständen schaden, zu hohe Kosten verursachen und/oder nicht umweltverträglich sind. Dies gilt vor allem für die genannte ETO-Sterilisation.

Des Weiteren ist es bekannt, die Sterilisation unter Zuhilfenahme von Ozon durchzuführen. Dies hat insbesondere gegenüber der ETO-Sterilisation den Vorteil, dass das Ozon nach erfolgter Sterilisation über einen Katalysator zu Sauerstoff und Wasserstoff umgesetzt und damit unschädlich gemacht werden kann. Sterilisationsverfahren, die unter dem Einsatz von Ozon arbeiten, sind aus dem Stand der Technik ebenfalls bekannt.

WO0158499 offenbart ein Desinfektionsverfahren mit Ozon und Wasser, wobei diese wiederholt in eine Behandlungskammer gesprüht werden.

So ist es beispielsweise aus der EP 1 175 230 B1 sowie aus der EP 1 455 843 B1 bekannt, einen Sterilisationsvorgang derart vorzunehmen, dass nach der Evakuierung einer Sterilisationskammer die Befeuchtung der in der Sterilisationskammer befindlichen Atmosphäre und sodann die Befüllung mit Ozon erfolgt, wodurch der Sterilisierungsvorgang eingeleitet wird. Diese Behandlungsschritte werden wiederholt, bevor die Sterilisationskammer gespült und schließlich geöffnet wird, so dass die sterilisierten Gegenstände entnommen werden können. Die aus den genannten Druckschriften bekannten Vorrichtungen und Verfahren eignen sich in der Praxis wegen ihres Aufbaus und der Verfahrensdurchführung nur für kleine Gebinde, wie beispielsweise Operationsbesteck in kleinen Behältern. Des Weiteren sieht das aus der EP 1 455 843 B1 bekannte Verfahren vor, dass die Temperatur der zu sterilisierenden Gegenstände zunächst der Temperatur der Atmosphäre in der Sterilisationskammer angeglichen wird, was insbesondere bei größeren Gebinden sehr lange dauern würde und daher nachteilig ist.

EP 0 016 887 A1 offenbart eine Sterilisationsvorrichtung mit einer Sterilisationskammer, in die die zu sterilisierenden Gegenstände eingelegt werden. Zum Sterilisieren wird die Sterilisationskammer evakuiert und ein Sterilisationsmittel eingeführt. Dabei wird in der Sterilisationskammer ein Überdruck erzeugt. Als Sterilisierungsmittel wird ein Biozid-Gas, wie beispielsweise Ethylenoxid, eingesetzt.

Der vorliegenden Erfindung liegt die Aufgabe zugrunde, ein Verfahren zur Sterilisation eines oder mehrerer Gegenstände bereitzustellen, mit dem auch größere Einheiten bzw. eine Vielzahl von Gegenständen effizient sterilisiert werden können.

Diese Aufgabe wird durch ein Verfahren mit den Merkmalen des Anspruchs 1 sowie durch eine Vorrichtung mit den Merkmalen des Anspruchs 10 gelöst.

Es ist vorgesehen, dass der oder die Gegenstände zunächst in eine Sterilisationskammer eingelegt werden, dass sodann das Evakuieren und Befeuchten der Sterilisationskammer bzw. der darin befindlichen Atmosphäre erfolgt. Anschließend erfolgt das Einführen eines Sterilisationsmittels in die Sterilisationskammer sowie die Erzeugung eines über dem Atmosphärendruck liegenden Überdruckes in der Sterilisationskammer. Dieser Überdruck wird dann unmittelbar nach Erreichen eines Überdruckwertes wieder abgesenkt oder auch für eine Zeitspanne gehalten, was eine bevorzugte Ausgestaltung der Erfindung darstellt. Die Schritte des Evakuierens und Befeuchtens, des Einführens eines Sterilisationsmittels, die Erzeugung des genannten Überdruckes sowie des Haltens eines Überdruckes werden einmal oder mehrfach wiederholt.

Die Erzeugung eines über dem Normaldruck liegenden Überdruckes wenigstens für einen Zeitpunkt und bevorzugt für eine bestimmte Zeitspanne bringt den Vorteil mit sich, dass das Sterilisationsmittel aktiv auf die Oberfläche des oder der zu sterilisierenden Gegenstände und insbesondere auch in das Lumen des Sterilisationsgutes gepresst wird. Dadurch lässt sich eine besonders effiziente Sterilisation auch größerer und/oder komplexerer Einheiten vornehmen. Eine Temperaturanpassung dahingehend, dass die Temperatur des Sterilisiergutes an die der Atmosphäre in der Sterilisationskammer angeglichen werden muss, ist nicht zwingend erforderlich und wird vorteilhaft auch nicht durchgeführt. Grundsätzlich ist es jedoch auch nicht ausgeschlossen, diesen Verfahrensschritt zusätzlich vorzusehen.

Wesentlich ist, dass wenigstens für einen Augenblick und besonders bevorzugt für eine gewisse Zeitspanne ein Überdruck in der Sterilisationskammer gehalten wird, der über dem Atmosphärendruck liegt. Auf diese Weise lässt sich auch Sterilisationsgut sterilisieren, das sich in einer Umverpackung befindet und/oder Sterilisationsgut, das ein Lumen aufweist, wie z. B. Schläuche.

Die Erzeugung des Überdruckes kann beispielsweise durch die Zufuhr eines Stützgases bewirkt werden. In bevorzugter Ausgestaltung erfolgt die Herstellung eines Überdruckes vergleichsweise schnell, wodurch das Sterilisationsmittel aktiv in ein Lumen, insbesondere in ein Schlauchlumen gepresst wird.

Nach der Druckzunahme erfolgt die Entspannung, was zum Ausfallen der während der Atmosphäre gelösten Flüssigkeit führt. Hierdurch entsteht eine nebelartige Atmosphäre, die als Aerosol zusätzlich Wirkung zeigt. Diese kann erzeugt werden durch schnelles Abkühlen eines gesättigten Gases oder auch durch schnelles Entspannen, wobei es sich bei der letzteren Variante um eine bevorzugte Ausgestaltung der Erfindung handelt.

Die erfindungsgemäßen Druckwechsel können also eine weitere zusätzliche Befeuchtung herbeiführen, nämlich einerseits durch erzwungene Kondensation durch vergleichsweise schnelle Erhöhung des Druckes gemäß Schritt d) in Anspruch 1 sowie durch Ausfallen der gelösten Flüssigkeit beim schnellen Entspannen durch Auflösen des Überdrucks bzw. durch Evakuieren gemäß Schritt b) in Anspruch 1.

In einer bevorzugten Ausführung ist ein Verfahren zur Sterilisation eines oder mehrerer Gegenstände vorgesehen, bei dem der oder die Gegenstände in eine Sterilisationskammer eingelegt werden, bei dem vorher, gleichzeitig oder anschließend ein Aerosol erzeugt wird, das das Sterilisationsmittel enthält oder aus diesem besteht und bei dem sodann dieses Sterilisationsmittel zumindest teilweise in Form eines Aerosols der Sterilisationskammer zugeführt wird.

Die Sterilisation mittels eines Aerosols stellt eine besonders effektive bzw. wirksame Methode dar, den oder die Gegenstände, die sich in der Sterilisationskammer befinden, zu sterilisieren.

Dem Einführen eines Sterilisationsmittels in Form eines Aerosols in die Sterilisationskammer kann der Schritt des Evakuierens und Befeuchtens der Sterilisationskammer vorausgehen. Wie oben ausgeführt, kann die Befeuchtung dadurch erfolgen, dass eine Flüssigkeit, insbesondere ein Gemisch enthaltend Wasser und/oder Wasserstoffperoxid, vorzugsweise durch Anlegen eines Vakuums zum Verdampfen gebracht wird und die verdampfte Flüssigkeit der Sterilisationskammer zugeführt wird.

In weiterer Ausgestaltung der Erfindung kann vorgesehen sein, dass das Sterilisationsmittel nach einer Einwirkzeit wieder entfernt wird und sodann neues Sterilisationsmittel in Form eines Aerosols zugeführt wird. Diese Abfolge kann ein- oder mehrfach wiederholt werden.

Besonders vorteilhaft, jedoch nicht zwingend, ist es in dieser Ausgestaltung, wenn in der Sterilisationskammer ein über Atmosphärendruck liegender Überdruck erzeugt wird. Dieser kann für einen Zeitpunkt oder eine Zeitspanne gehalten werden. Auch in dieser Ausgestaltung ist es möglich, einen oder mehrere der Verfahrensschritte ein- oder mehrfach zu wiederholen.

Die Erzeugung des Aerosols kann beispielsweise durch einen Ultraschallzerstäuber, einen Ultraschallvernebler, eine Einspritzdüse oder durch eine sogenannte Laskindüse erfolgen, wie dies unten näher erläutert wird.

In einer bevorzugten Ausgestaltung der Erfindung ist vorgesehen, dass der Überdruck gemäß Schritt d) in Anspruch 1 bei einem Wert von > 1.200 mbar, vorzugsweise bei einem Wert von > 1.500 mbar und besonders bevorzugt bei einem Wert von > 2.000 mbar liegt.

Während, insbesondere während Schritt e), des Haltens eines Überdruckes für eine Zeitspanne kann der Druckwert derart eingestellt sein, dass er während eines Teils der Zeitspanne oder während der gesamten Zeitspanne über Atmosphärendruck liegt. Bevorzugt ist es, wenn der Druck während der Zeitspanne auf demselben Wert oder in etwa auf demselben Wert liegt, wie nach der Erzeugung des Überdruckes gemäß Schritt d) gemäß Anspruch 1. Grundsätzlich ist es auch denkbar, dass dieser Druckwert nicht gehalten wird, sondern beispielsweise ein fallender Druck während der Zeitspanne in Schritt e) gemäß Anspruch 1 erfolgt.

In weiterer Ausgestaltung der Erfindung ist vorgesehen, dass die Befeuchtung der Sterilisationskammer beispielsweise gemäß Schritt b) in Anspruch 1 dadurch erfolgt, dass eine Flüssigkeit, insbesondere ein Gemisch enthaltend Wasser und/oder Wasserstoffperoxid, durch Anlegen eines Vakuums, insbesondere durch das Evakuieren beispielsweise gemäß Schritt b) in Anspruch 1 zum Verdampfen gebracht wird und die verdampfte Flüssigkeit der Sterilisationskammer zugeführt wird.

Denkbar ist es somit, mit einer Vakuumpumpe die Sterilisationskammer zu evakuieren und dabei auch gleich oder anschließend das Verdampfen der Flüssigkeit zu leiten. Befindet sich die Flüssigkeit in einem Behälter, vorzugsweise in einem Aerosolbehälter, der mit der Sterilisationskammer verbunden ist, wird in dem Moment, in dem der Dampfdruck der Flüssigkeit in dem Aerosolbehälter erreicht ist, kein oder kaum noch weiterer Unterdruck erzeugt, da es zu einem Verdampfen der Flüssigkeit in dem Aerosolbehälter kommt. Ist somit der Dampfdruck der Flüssigkeit in dem Behälter erreicht, fängt diese Flüssigkeit an zu verdampfen und gelangt auf diese Weise in die Sterilisationskammer, in der sie zu einer Befeuchtung der darin befindlichen Atmosphäre führt.

Die Vakuumpumpe wird in diesem Fall somit nicht nur zum Evakuieren der Sterilisationskammer, sondern auch zum Verdampfen der Flüssigkeit in dem Behälter und somit auch zur Befeuchtung der Sterilisationskammer benutzt.

In weiterer Ausgestaltung der Erfindung ist vorgesehen, dass das Sterilisationsmittel in die Sterilisationskammer in Form eines Aerosols eingeführt wird, das Ozon und Wasserstoffperoxid und alternativ oder zusätzlich zu Wasserstoffperoxid/Wasser oder eines oder mehrerer der Reaktionsprodukte dieser Substanzen enthält. Dabei ist es besonders bevorzugt, wenn das Aerosol durch Einleiten von Ozon in eine Flüssigkeit entsteht, die Wasser und/oder Wasserstoffperoxid enthält.

Denkbar ist es beispielsweise, Ozongas durch eine sogenannte Laskindüse zu leiten. Diese Düse ist mit ihrer Auslassöffnung unterhalb des Pegels einer Flüssigkeit angeordnet, die aus Wasserstoffperoxid und/oder Wasser besteht oder eine oder mehrerer dieser Substanzen enthält. In der entstehenden, aus Ozon bestehenden Gasblase befinden sich kleine Flüssigkeitstropfen, die insgesamt eine sehr große Oberfläche haben, die direkt mit dem Ozongas in Berührung stehen. Der Kontakt des Ozons mit Wasser und/oder mit Wasserstoffperoxid führt zur Aktivierung der Flüssigkeiten durch eine Reaktion zwischen Wasser und/oder Wasserstoffperoxid und dem Ozon. Diese Reaktion führt zum Aufspalten des Wassers / Wasserstoffperoxids und dann zur Radikalbildung. Die Hydroxidradikale OH sind reaktiv und aggressiv und bewirken durch Töten von Mikroorganismen den gewünschten Sterilisationserfolg.

Aufgrund der vergleichsweise großen Kontaktfläche zwischen der Flüssigkeit mit dem Ozongas werden viele der genannten Reaktionen angeregt, so dass eine entsprechend hohe Ausbeute an OH·-Radikalen vorliegt.

In weiterer Ausgestaltung der Erfindung ist vorgesehen, dass das Einführen eines Sterilisationsmittels in die Sterilisationskammer bei einem Druck erfolgt, der unterhalb des Atmosphärendruckes liegt.

Denkbar ist es somit beispielsweise, die Sterilisationskammer durch Aerosol zu befüllen, so lange der Druck unterhalb des Atmosphärendruckes liegt, so dass der Einführvorgang des Sterilisationsmittels abgeschlossen ist, bevor in der Sterilisationskammer Atmosphärendruck erreicht wird. Denkbar ist es, dass Einführen der Sterilisationsmittel bei einem Druck bis < 200 mbar, vorzugsweise bis < 100 mbar durchzuführen.

Diese Vorgehensweise ermöglicht es, den Aerosolbehälter und/oder -generator während des gesamten Betriebes stets bei einem Druckwert zu halten, der unterhalb des Atmosphärendruckes liegt. Diese Vorgehensweise bringt den Vorteil mit sich, dass ein Nachfüllen des Aerosolbehälters durch Wasserstoffperoxid bzw. Wasser besonders einfach ist, da lediglich ein Dosierventil geöffnet werden muss, durch das dann die entsprechende Flüssigkeit nachgezogen werden kann.

In weiterer Ausgestaltung der Erfindung ist vorgesehen, dass sich an Schritt f) gemäß Anspruch 1 das Spülen und Entgasen der Sterilisationskammer anschließt, wobei des Spülen und Entgasen ein- oder mehrfach durchgeführt wird.

Denkbar ist es, diese Spül- und Entgasungsphase durch Wiederholungen von Evakuierungen und Belüftungen auszugestalten. Ebenfalls ist es denkbar, eine Trockenphase vorzusehen, die vorzugsweise im evakuierten Zustand der Sterilisationskammer durchgeführt wird. Sodann kann der Sterilisationsvorgang abgeschlossen werden.

In weiterer Ausgestaltung der Erfindung ist vorgesehen, dass das Halten des Überdruckes insbesondere gemäß Schritt e) gemäß Anspruch 1 für eine Zeitspanne erfolgt, deren Dauer im Bereich < 10 min. vorzugweise < 5 min. und besonders bevorzugt < 2 min. liegt.

Weiterhin kann vorgesehen sein, dass die Zeitspanne zwischen zwei aufeinanderfolgenden Druckerhöhungen gemäß Schritt d) gemäß Anspruch 1 im Bereich < 20 min., vorzugsweise < 15 min. und besonders bevorzugt < 10 min. liegt.

Die vorliegende Erfindung betrifft des Weiteren eine Sterilisationsvorrichtung mit den Merkmalen des Anspruchs 10.

Danach ist vorgesehen, dass die Sterilisationsvorrichtung eine Sterilisationskammer sowie eine Einheit zur Erzeugung eines Sterilisationsmittels aufweist. Des Weiteren sind Mittel zur Zufuhr des Sterilisationsmittels in die Sterilisationskammer vorgesehen.

Die Sterilisationsvorrichtung weist erste Mittel zur Evakuierung und Befeuchtung der Sterilisationskammer, zweite Mittel zur Zufuhr des Sterilisationsmittels in die Sterilisationskammer, dritte Mittel zur Erzeugung eines über Atmosphärendruckes liegenden Überdruckes in der Sterilisationskammer sowie vierte Mittel zum Halten eines Überdruckes in der Sterilisationskammer für einen Zeitpunkt oder für eine Zeitspanne auf. Des Weiteren weist die Sterilisationsvorrichtung eine Steuer- oder Regelungseinheit auf, die derart ausgeführt ist, dass diese die genannten Mittel derart ansteuert, dass die durch die Mittel ausgeführten Schritte wenigstens einfach oder mehrfach wiederholt werden.

In einer bevorzugten Ausführung weist die Sterilisationsvorrichtung eine Sterilisationskammer mit einer Einheit zur Erzeugung des Sterilisationsmittels sowie zur Zufuhr des Sterilisationsmittels in die Sterilisationskammer aufweist. Dabei ist die Einheit zur Erzeugung des Sterilisationsmittels und/oder zur Zufuhr des Sterilisationsmittels in die Sterilisationskammer derart ausgeführt, dass das Sterilisationsmittel zumindest teilweise in Form eines Aerosols vorliegt.

Nach dem Einführen des Sterilisationsmittels in die Sterilisationskammer kann ein überdruck in der Sterilisationskammer erzeugt werden, wobei dies für diese Ausgestaltung der Erfindung jedoch kein zwingendes Merkmal darstellt.

In bevorzugter Ausgestaltung der Erfindung ist vorgesehen, dass die ersten und/oder die zweiten Mittel eine Vakuumpumpe und einen Aerosolbehälter und/oder -generator aufweisen und/oder dass die dritten Mittel eine Quelle oder einen Anschluß für ein unter Druck stehendes Gas und/oder dass die vierten Mittel ein oder mehrere Ventile zum Abschließen der Sterilisationskammer umfassen.

Die vierten Mittel können somit durch Ventile gebildet werden, die die Sterilisationskammer abschließen, so dass der erzeugte Überdruck während einer gewünschten Zeitspanne gehalten werden kann.

Weiterhin kann vorgesehen sein, dass Aerosolbehälter bzw. -generator der Sterilisationskammer vorgeschaltet und die Vakuumpumpe der Sterilisationskammer nachgeschaltet ist und dass die Steuer- oder Regelungseinheit die ersten Mittel derart ansteuert, dass bei geöffneten Leitungen zwischen Aerosolbehälter und Sterilisationskammer und zwischen Sterilisationskammer und Vakuumpumpe zunächst das Evakuieren und sodann ohne Änderung der Betriebsweise der Vakuumpumpe das Befeuchten der Sterilisationskammer erfolgt. Dies kann dadurch erreicht werden, dass die Vakuumpumpe zunächst das Evakuieren der Sterilisationskammer bewirkt und sodann aufgrund des verringerten Gesamtdruckes es zu einem Verdampfen der Flüssigkeit in dem Aerosolbehälter kommt, wodurch sodann die Sterilisationskammer bzw. die darin befindliche Atmosphäre befeuchtet wird.

In weiterer Ausgestaltung der Erfindung ist vorgesehen, dass die Einheit zur Erzeugung des Sterilisationsmittels und/oder die ersten Mittel einen Aerosolbehälter bzw. -generator aufweisen, in dem sich eine Flüssigkeit befindet, die Wasser und/oder Wasserstoffperoxid enthält oder daraus besteht, und in dem sich eine Düse befindet, deren wenigstens eine Auslassöffnung unter dem Pegel der Flüssigkeit liegt und durch die Ozon in die Flüssigkeit eingeleitet wird.

In weiterer Ausgestaltung der Erfindung ist vorgesehen, dass die Sterilisationskammer über einen ersten Anschluß verfügt, der mit einer Gasleitung zur Erzeugung des Überdruckes in Verbindung steht, die ihrerseits mit einer Gasquelle für ein Stützgas in Verbindung steht. Des Weiteren verfügt die Sterilisationskammer in diesem Ausführungsbeispiel über einen zweiten Anschluß, der mit einer von einem Aerosolbehälter führenden Leitung in Verbindung steht, durch die das Aerosol nach seiner Herstellung in die Sterilisationskammer eingeführt werden kann.

Die Sterilisationskammer verfügt des Weiteren über einen dritten Anschluß, über den die Sterilisationskammer mit einer Vakuumpumpe in Verbindung steht.

Ferner ist es beispielsweise denkbar, das Sterilisationsmittel in Form eines Aerosols vorzulegen und dieses dann mit oder ohne Überdruck auf den oder die zu sterilisierenden Gegenstände einwirken zu lassen.

Weitere Einzelheiten und Vorteile der Erfindung werden anhand eines in der Zeichnung dargestellten Ausführungsbeispiels näher erläutert. Es zeigen:
- Figur 1:: eine schematische Darstellung einer Sterilisationsvorrichtung gemäß der vorliegenden Erfindung,
- Figur 2:: eine schematische Darstellung des Verlaufes der Temperatur sowie des Druckes in der Sterilisationskammer sowie der Temperatur des Aerosols während einer Behandlung,
- Figur 3:: eine vergrößerte Darstellung des Druckverlaufes in der Sterilisationskammer während eines Sterilisationszyklus,
- Figur 4:: eine schematische Ansicht eines Aerosolbehälters sowie der Düse zum Einleiten von Ozon in eine Mischung aus Wasserstoffperoxid und Wasser und
- Figur 5:: eine schematische Darstellung des Verlaufes der relativen Luftfeuchtigkeit und dem Druck in der Sterilisationskammer während der Behandlung.

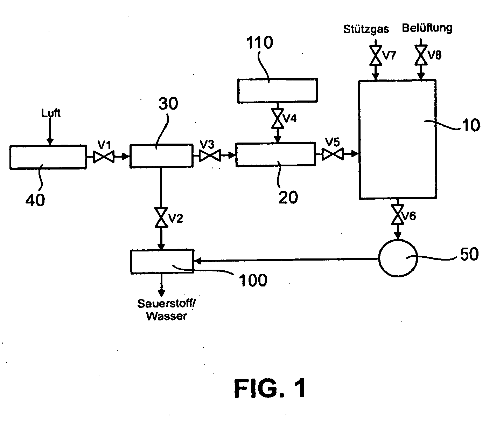

Figur 1 zeigt ein Blockschaltbild für ein Ausführungsbeispiel einer Sterilisationsvorrichtung gemäß der vorliegenden Erfindung.

Mit dem Bezugszeichen 10 ist eine Sterilisationskammer gekennzeichnet, die beispielsweise ein Volumen von bis zu 1 m³ haben kann sowie eine Tür, die sowohl zum Be- als auch zum Entladen dient.

Die zu sterilisierenden Gegenstände können beispielsweise auf Stahlkörben auf einer oder mehreren Ebenen in die Sterilisationskammer eingebracht werden.

Je nach Produktgröße können 150 bis 200 Produkte gleichzeitig sterilisiert werden.

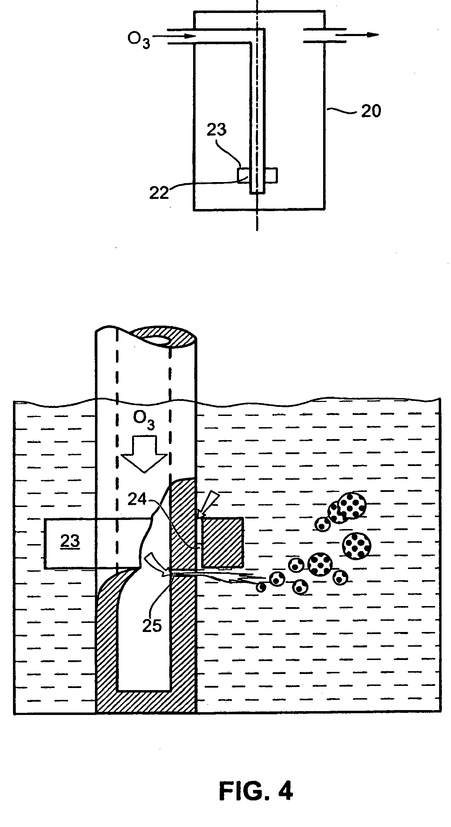

Der Sterilisationskammer vorgeschaltet ist ein Aerosolbehälter bzw. Aerosolgenerator 20. Einen exemplarischen Aufbau für einen solchen Aerosolgenerator zeigt Figur 4.

Dabei ist in Figur 4, obere Darstellung, gekennzeichnet, dass das Ozon aus dem Ozongenerator 30 gemäß Figur 1 über eine Leitung in einen unteren Bereich des Aerosolgenerators 20 gelangt. Dabei ist eine Düse 22 vorgesehen, die in vergrößerter Darstellung in Figur 4, untere Darstellung erkennbar ist. Wie dies aus Figur 4, untere Darstellung hervorgeht, gelangt Ozon in eine Flüssigkeit bestehend aus Wasser / Wasserstoffperoxid. Die Düse 22 sitzt unterhalb des Flüssigkeitsspiegels.

Wie dies aus Figur 4, untere Darstellung erkennbar ist, sind in einem kragenförmigen Bereich 23 eine oder mehrere Bohrungen 24 angeordnet. Direkt unter dieser bzw. diesen Bohrungen 24 ist in dem Düsenkörper wenigsten ein weiteres Loch 25 angeordnet, das unter dem unteren Endabschnitt der Bohrung 24 angeordnet ist. Ozon strömt aus dem Loch 25 und strömt an der vertikalen Bohrung 24 vorbei. Dadurch entsteht ein Unterdruck in der Bohrung 24, wodurch Flüssigkeit aus der Bohrung 24 in den austretenden Gasstrom gezogen wird. Dieser austretende Gasstrom verwirbelt und schert die Flüssigkeit derart, dass sich kleine Tropfen in der Gasblase bilden, wie dies in Figur 4, untere Darstellung, angedeutet ist. Die Gasblase steigt nach oben an die Flüssigkeitsoberfläche und gibt diese winzigen Tropfen frei, wodurch ein Aerosol gebildet wird. Diese Tropfen werden mit dem Gasstrom, der aus dem Flüssigkeitsbehälter 20 strömt, mitgerissen und gelangen über ein Rohrsystem in die dem Aerosolgenerator 20 nachgeschaltete Sterilisationskammer 10.

Wie dies weiter aus Figur 1 hervorgeht, ist dem Ozongenerator 30 ein Sauerstoffgenerator 40 vorgeschaltet, in dem 95%iger Sauerstoff aus der Umgebungsluft gewonnen wird. Hierzu dient beispielsweise ein Molekularsieb bzw. Zeolith. In dem Ozongenerator 30 wird der Sauerstoff in Ozon umgewandelt, was beispielsweise durch eine dielektrische Barriereentladung erfolgen kann.

Mit dem Bezugszeichen 100 in Figur 1 ist ein Katalysator gekennzeichnet, der geeignet ist, das Sterilisationsmittel nach dessen Gebrauch, insbesondere Wasserstoffperoxid und/oder Ozon bzw. deren Reaktionsprodukte zu zersetzen. Als Abgas entsteht dann lediglich Wasser und Sauerstoff. Bei dem Katalysator 100 kann es sich beispielsweise um Mangandioxid handeln.

Zur Erzeugung des gewünschten Vakuums in der Sterilisationskammer 10 ist dieser eine Vakuumpumpe 50 nachgeschaltet, die bei geöffnetem Ventil V6 und bei in Betrieb befindlicher Vakuumpumpe 50 zu einem Evakuieren der Sterilisationskammer 10 führt.

Mit dem Bezugszeichen 110 ist schließlich eine Einheit angedeutet, die der Zudosierung von Wasser und/oder Wasserstoffperoxid in den Aerosolgenerator 20 dient.

### Der Sterilisationsvorgang gestaltet sich im Einzelnen wie folgt:

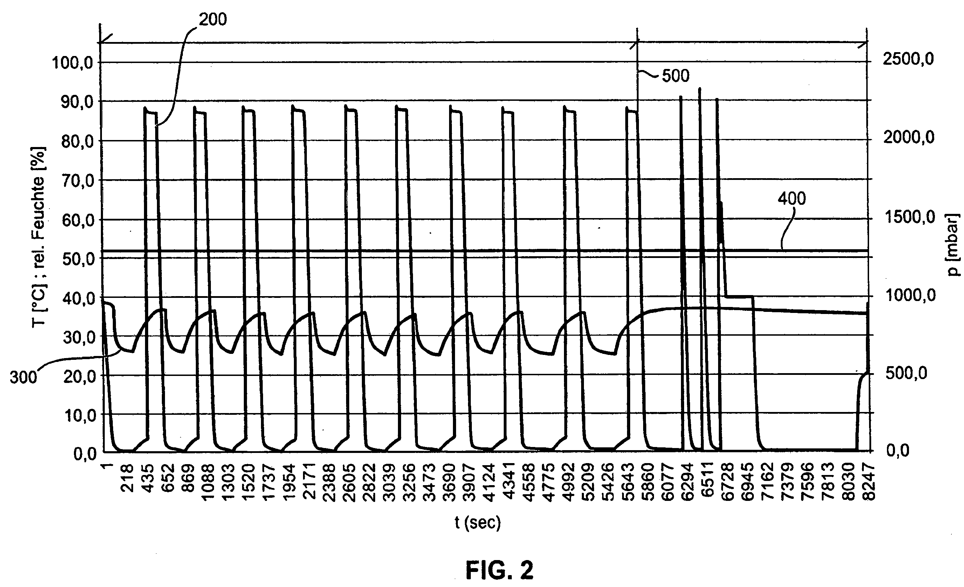

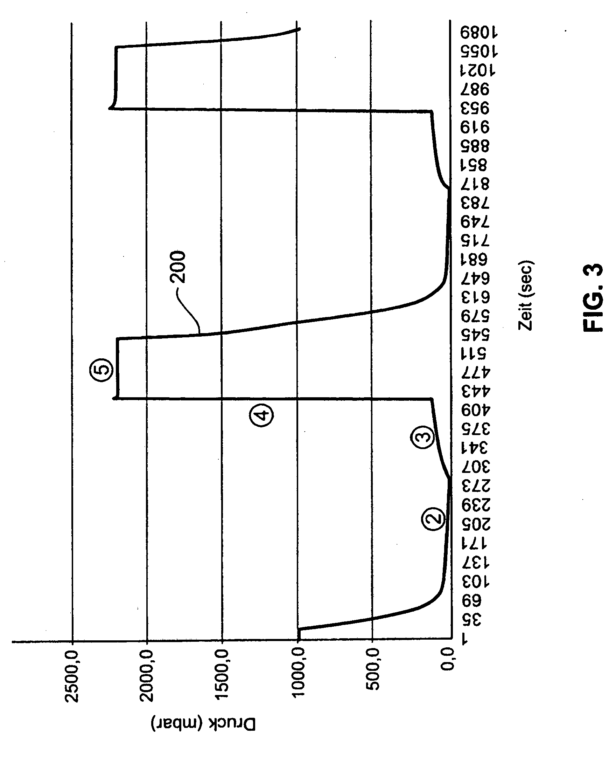

Nach dem Einlegen des oder der Gegenstände in die Sterilisationskammer 10 wird in der Sterilisationskammer 10 ein Vakuum erzeugt, wozu das Ventil V5 und V6 gemäß Figur 1 geöffnet und alle weiteren Ventile geschlossen werden. Die Vakuumerzeugung führt zu einer Reduzierung des Kammerdruckes, der in Figur 2 und Figur 3 mit dem Bezugszeichen 200 gekennzeichnet ist. Das Bezugszeichen 300 gemäß Figur 2 kennzeichnet die Temperatur des Aerosols und das Bezugszeichen 400 gemäß Figur 2 die Temperatur innerhalb der Sterilisationskammer 10.

Zur weiteren Erläuterung des Sterilisationsvorgangs wird auf Figur 3 verwiesen.

Durch das Evakuieren der Sterilisationskammer 10 mittels der Vakuumpumpe 50 fällt der Druck in der Kammer 10 vorzugsweise auf einen Wert < 10 mbar ab. Aufgrund der Tatsache, dass während des Evakuierungsvorgangs nicht nur das Ventil V6 in der Leitung zwischen Sterilisationskammer 10 und Vakuumpumpe 50, sondern auch das Ventil V5 in der Leitung zwischen dem Aerosolgenerator 20 und der Sterilisationskammer 10 geöffnet ist, kommt es nicht nur zu einer Evakuierung der Sterilisationskammer 10, sondern auch zu einem Verdampfen der Flüssigkeit in dem Aerosolbehälter 20, sofern deren Dampfdruck erreicht wird. Ist der Dampfdruck der Flüssigkeit, d. h. der Mischung aus Wasser und Wasserstoffperoxid in dem Aerosolbehälter erreicht, fängt diese an zu verdampfen und gelangt auf diese Weise in die Sterilisationskammer 10. Dieser Schritt ist in Figur 2 mit dem Bezugszeichen 2 gekennzeichnet. Die Vakuumpumpe 50 wird somit nicht nur zum Evakuieren, sondern auch zum Verdampfen der Flüssigkeit in dem Aerosolbehälter 20 genutzt.

Nach dieser Evakuierungsphase 2 wird aktiv Sterilisationsmittel in die Sterilisationskammer 10 eingebracht. Diese Phase ist in Figur 3 mit dem Bezugszeichen 3 gekennzeichnet. Wie dies im Einzelnen zu Figur 4 erläutert wurde, vermischt sich durch das Laskinprinzip das Ozon mit Wasserstoffperoxid / Wasser. Durch den Kontakt des Ozons mit Wasser und/oder Wasserstoffperoxid kommt es zur Entstehung von Hydroxidradikalen, die mit dem Aerosol in die Sterilisationskammer 10 gelangen und dort zum Sterilisationsprozess beitragen bzw. die maßgeblichen Sterilisationsmittel darstellen.

Während der Phase 3 gemäß Figur 3 sind die Ventile V3 und V5 geöffnet und alle weiteren Ventile geschlossen. Das ozonhaltige Aerosol gelangt aufgrund des Druckgefälles zwischen Ozongenerator 30 und Sterilisationskammer 10 in die Sterilisationskammer 10. Dadurch kommt es zu einem Druckanstieg, wie dies aus Figur 3 ersichtlich ist. Dieser Druckanstieg ist ein Maß für die Konzentration des Sterilisationsmittels in der Sterilisationskammer. Hierbei wird die Konzentration auf ein für das zu sterilisierende Gut optimalen Wert eingestellt. Nach dem Einführen des Sterilisationsmittels wird der Druck in der Sterilisationskammer 10 mittels eines Stützgases auf einen Wert oberhalb des Atmosphärendruckes erhöht, wie dies in Schritt 4 gemäß Figur 3 dargestellt ist. In dem dort dargestellten Ausführungsbeispiel wird der Druck auf einen Wert von ca. 2,25 bar erhöht. Dieses Stützgas gelangt dadurch in die Sterilisationskammer 10, wofür das Ventil 7 gemäß Figur 1 für eine vorbestimmte Zeitspanne oder bis zum Erreichen eines bestimmten Druckwertes geöffnet wird.

Während dieser Phase sind alle weiteren Ventile der Vorrichtung geschlossen.

Wie dies weiter aus Figur 3 hervorgeht, wird der Überdruck in der Sterilisationskammer 10 für eine bestimmte Zeitspanne gehalten. Diese Phase ist in Figur 3 mit dem Bezugszeichen 5 gekennzeichnet. Diese Phase kann beispielsweise bei einer Minute bzw. im Minutenbereich liegen.

Aufgrund dieses vergleichsweise hohen Druckes in der Sterilisationskammer gelingt es, das Sterilisationsgut effektiv zu sterilisieren. Dies gilt beispielsweise für Sterilisationsgut, das ein Lumen aufweist, wie beispielsweise Schläuche und/oder für Sterilisationsgut, das sich in einer Umverpackung befindet.

Nach dem Halten des Überdruckes für eine bestimmte Zeitspanne gemäß Schritt 5 in Figur 3 wird der Prozesse wiederholt, d. h. es wird evakuiert und befeuchtet, dann das Sterilisationsmittel eingeführt und sodann wieder der Überdruck erzeugt.

Aufgrund der Tatsache, dass das Befüllen der Sterilisationskammer 10 mit Sterilisationsmittel gemäß Schritt 3 in Figur 3 beendet wird, wenn in der Sterilisationskammer 10 noch ein erheblicher Unterdruck herrscht, kann erreicht werden, dass in dem Aerosolgenerator 20 stets ein Unterdruck herrscht. Dies ermöglicht es, dass durch Öffnen des Ventils V 4 aus einem Reservoir 110 ohne Weiteres Flüssigkeit, d. h. Wasser, Wasserstoffperoxid und/oder eine Mischung aus beiden Substanzen nachgezogen werden kann.

Wie dies weiter aus Figur 1 hervorgeht, steht die Pumpe 50 auslassseitig mit dem Katalysator 100 in Verbindung, so dass das auf der Druckseite der Pumpe 50 anfallende Gas in dem Katalysator 100 zersetzt werden kann.

Wie dies aus Figur 2 hervorgeht, kann der oben beschriebene Ablauf des Sterilisationsvorganges mehrfach wiederholt werden. In dem in Figur 2 dargestellten Beispiel handelt es sich um 10 aufeinander folgende Sterilisationsschritte.

Danach schließt sich in dem hier dargestellten Ausführungsbeispiel das Spülen und Entgasen der Sterilisationskammer 10 durch mehrmaliges Evakuieren und Belüften an. Diese Phase ist in Figur 2 rechts neben dem vertikalen Strich 500 gekennzeichnet, der die Sterilisationsphase von der Spül- und Entgasungsphase trennt.

Während der Spül- und Entgasungsphase kommt es mehrfach zu einem Evakuieren und erneutem Unterdrucksetzen sowie zu einem Belüften der Sterilisationskammer 10. An diese Phase kann sich eine Trockenphase anschließen, in der sich in der Sterilisationskammer 10 vorzugsweise Vakuum befindet.

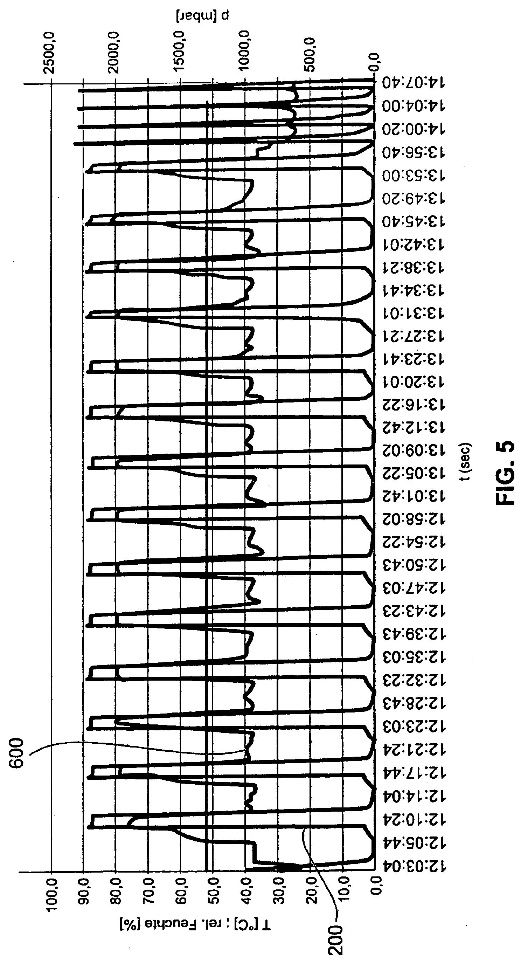

Figur 5 zeigt schließlich den zeitlichen Verlauf des Druckes 200 in der Sterilisationskammer 10 sowie parallel dazu die relative Luftfeuchtigkeit 600 in der Sterilisationskammer.

Aus Figur 5 ergibt sich, dass die relative Luftfeuchtigkeit 600 den Verlauf des Druckes 200 in der Sterilisationskammer 10 folgt. Bei schnellem Stützgaseinlas in die Sterilisationskammer 10 wird die in dem Gas befindliche gelöste Flüssigkeit gezwungen zu kondensieren, so dass ein in der Kammer befindlicher Luftfeuchtefühler einen vergleichsweise hohen Wert anzeigt. Das nachfolgende Evakuieren führt zu einem Ausfallen von Flüssigkeit aus dem Gas (Nebelbildung), was zu einem Absinken der absoluten sowie auch der relativen Feuchtigkeit führt.

## Patentansprüche

1. Verfahren zur Sterilisation eines oder mehrerer Gegenstände umfassend die Schritte:
a) Einlegen des oder der Gegenstände in eine Sterilisationskammer (10),
b) Evakuieren und Befeuchten der Sterilisationskammer (10),
c) Einführen eines Sterilisationsmittels in die Sterilisationskammer (10),
d) Erzeugung eines über Atmosphärendruckes liegenden Überdruckes in der Sterilisationskammer (10)
e) Halten eines Überdruckes in der Sterilisationskammer (10) für eine Zeitspanne,
f) ein- oder mehrfaches Wiederholen der Schritte b) - e), wobei
das Sterilisationsmittel in die Sterilisationskammer (10) in Form eines Aerosols eingeführt wird und Ozon und Wasserstoffperoxid und/oder Wasser oder eines oder mehrerer der Reaktionsprodukte dieser Substanzen enthält, und
das Aerosol durch Einleiten von Ozon in eine Flüssigkeit entsteht, die Wasser und/oder Wasserstoffperoxid enthält.

2. Verfahren nach Anspruch 1, **dadurch gekennzeichnet, dass** der Überdruck insbesondere gemäß Schritt d) bei einem Wert > 1.200 mbar, vorzugsweise bei einem Wert > 1.500 mbar und besonders bevorzugt bei einem Wert > 2.000 mbar liegt.

3. Verfahren nach Anspruch 1 oder 2, **dadurch gekennzeichnet, dass** der Überdruck insbesondere gemäß Schritt e) ein Druckwert darstellt, der während eines Teils der Zeitspanne oder während der gesamten Zeitspanne über Atmosphärendruck liegt und vorzugsweise dem Überdruck insbesondere gemäß Schritt d) entspricht.

4. Verfahren nach einem der vorhergehenden Ansprüche, **dadurch gekennzeichnet, dass** das Befeuchten der Sterilisationskammer (10) dadurch erfolgt, dass eine Flüssigkeit, insbesondere ein Gemisch enthaltend Wasser und Wasserstoffperoxid, durch Anlegen eines Vakuums, insbesondere durch das Evakuieren zum Verdampfen gebracht wird und die verdampfte Flüssigkeit der Sterilisationskammer (10) zugeführt wird.

5. Verfahren nach einem der vorhergehenden Ansprüche, **dadurch gekennzeichnet, dass** das Einführen eines Sterilisationsmittels in die Sterilisationskammer (10) bei einem Druck in der Sterilisationskammer erfolgt, der unterhalb des Atmosphärendruckes liegt.

6. Verfahren nach einem der vorhergehenden Ansprüche, **dadurch gekennzeichnet, dass** anschließend an Schritt f) gemäß Anspruch 1 bzw. nach Abschluß der Sterilisation das Spülen und Entgasen der Sterilisationskammer (10) durchgeführt wird, wobei des Spülen und Entgasen ein- oder mehrfach durchgeführt wird.

7. Verfahren nach einem der vorhergehenden Ansprüche, **dadurch gekennzeichnet, dass** das Halten des Überdruckes gemäß Schritt e) für eine Zeitspanne erfolgt, deren Dauer im Bereich < 10 min. liegt.

8. Verfahren nach einem der vorhergehenden Ansprüche, **dadurch gekennzeichnet, dass** die Zeitspanne zwischen zwei aufeinanderfolgenden Druckerhöhungen gemäß Schritt d) im Bereich < 20 min. liegt.

9. Sterilisationsvorrichtung mit einer Sterilisationskammer (10), mit
einer Einheit zur Erzeugung des Sterilisationsmittels sowie zur Zufuhr des Sterilisationsmittels in die Sterilisationskammer (10),
ersten Mitteln zur Evakuierung und Befeuchtung der Sterilisationskammer (10),
zweiten Mitteln zur Zufuhr des Sterilisationsmittels in die Sterilisationskammer (10),
dritten Mitteln zur Erzeugung eines über Atmosphärendruckes liegenden Überdruckes in der Sterilisationskammer (10), sowie
vierten Mitteln zum Halten eines Überdruckes in der Sterilisationskammer (10) für einen Zeitpunkt oder eine Zeitspanne, wobei
die Sterilisationsvorrichtung eine Steuer- oder Regelungseinheit aufweist, die dazu ausgelegt ist, die ersten bis vierten Mittel derart anzusteuern, dass die durch die ersten Mittel bewirkte Evakuierung und Befeuchtung, das durch die zweite Mittel bewirkte Zuführen des Sterilisationsmittels, das durch die dritten Mittel bewirkte Erzeugen eines über Atmosphärendruck liegenden Überdruckes sowie das durch die vierte Mittel bewirkte Halten eines Überdruckes für eine Zeitspanne ein- oder mehrfach wiederholt werden,
**dadurch gekennzeichnet, dass**
die Steuer- und Regelungseinheit zur Durchführung des Verfahrens nach Anspruch 1 ausgeführt ist.

10. Sterilisationsvorrichtung nach Anspruch 9, **dadurch gekennzeichnet, dass** mindestens eines der ersten und zweiten Mittel eine Vakuumpumpe (50) und einen Aerosolbehälter und/oder-generator (20) aufweist.

11. Sterilisationsvorrichtung nach Anspruch 10, **dadurch gekennzeichnet, dass** der Aerosolbehälter und/oder -generator (20) der Sterilisationskammer (10) vorgeschaltet und die Vakuumpumpe (50) der Sterilisationskammer (10) nachgeschaltet ist und dass die Steuer- oder Regelungseinheit die ersten Mittel derart ansteuert, dass bei geöffneten Leitungen zwischen Aerosolbehälter und/oder -generator (20) und Sterilisationskammer (10) und zwischen Sterilisationskammer (10) und Vakuumpumpe (50) zunächst das Evakuieren und ohne Änderung der Betriebsweise der Sterilisationsvorrichtung sodann das Befeuchten der Sterilisationskammer (10) erfolgt.

12. Sterilisationsvorrichtung nach einem der Ansprüche 9 bis 11, **dadurch gekennzeichnet, dass** die Sterilisationskammer (10) über einen ersten Anschluß verfügt, der mit einer Gasleitung zur Erzeugung des Überdruckes in Verbindung steht, über einen zweiten Anschluß verfügt, der mit einer von einem Aerosolbehälter führenden Leitung in Verbindung steht, und über einen dritten Anschluß verfügt, über den die Sterilisationskammer (10) mit einer Vakuumpumpe (50) in Verbindung steht.

13. Sterilisationsvorrichtung nach Anspruch 9, **dadurch gekennzeichnet, dass** die dritten Mittel ein Quelle für ein unter Druck stehendes Gas und/oder dass die vierten Mittel ein oder mehrere Ventile zum Abschließen der Sterilisationskammer (10) umfassen.

## Claims

1. A method of sterilizing one or more articles comprising the steps:
a) placing the article or articles into a sterilization chamber (10);
b) evacuating and humidifying the sterilization chamber (10);
c) introducing a sterilization agent into the sterilization chamber (10);
d) generating an excess pressure above atmospheric pressure in the sterilization chamber (10);
e) maintaining an excess pressure in the sterilization chamber (10) for a time period;
f) repeating steps b) - e) once or multiple times, with
the sterilization agent being introduced into the sterilization chamber (10) in the form of an aerosol and containing ozone and hydrogen peroxide and/or water or one or more of the reaction products of these substances; and
the aerosol arising by the introduction of ozone into a liquid which contains water and/or hydrogen peroxide.

2. A method in accordance with claim 1, **characterized in that**
the excess pressure, in particular in accordance with step d), lies at a value > 1,200 mbar, preferably at a value > 1,500 mbar, and particularly preferably at a value > 2,000 mbar.

3. A method in accordance with one of claims 1 or 2, **characterized in that** the excess pressure, in particular in accordance with step e), represents a pressure value which is above atmospheric pressure during some of the time period or during the total time period and which preferably corresponds to the excess pressure in particular in accordance with step d).

4. A method in accordance with one of the preceding claims, **characterized in that** the humidification of the sterilization chamber takes (10) place **in that** a liquid, in particular a mixture containing water and hydrogen peroxide, is vaporized by application of a vacuum, in particular by evacuation, and the vaporized liquid is supplied to the sterilization chamber (10).

5. A method in accordance with one of the preceding claims, **characterized in that** the introduction of a sterilization agent into the sterilization chamber (10) takes place at a pressure in the sterilization chamber which is below the atmospheric pressure.

6. A method in accordance with one of the preceding claims, **characterized in that** the purging and degassing of the sterilization chamber (10) is carried out subsequent to step f) in accordance with claim 1 or after the termination of the sterilization, with the purging and degassing being carried out once or a multiple of times.

7. A method in accordance with one of the preceding claims, **characterized in that** the maintaining of the excess pressure in accordance with step e) takes place for a time period whose duration is in the range < 10 min.

8. A method in accordance with one of the preceding claims, **characterized in that** the time period between two consecutive pressure increases in accordance with step d) is in the range < 20 min.

9. A sterilization apparatus having a sterilization chamber (10)
Having a unit for producing the sterilization agent and for supplying the sterilization agent into the sterilization chamber (10);
having first means for evacuating and humidifying the sterilization chamber (10);
having second means for supplying the sterilization agent into the sterilization chamber (10);
having third means for generating an excess pressure above atmospheric pressure in the sterilization chamber (10); and
having fourth means for maintaining an excess pressure in the sterilization chamber (10) for a point in time or for a time period, wherein
the sterilization apparatus has a control or regulation unit which is configured to control the first to fourth means such that the evacuation and humidification effected by the first means, the supply of the sterilization agent effected by the second means, the generation of an excess pressure above atmospheric pressure effected by the third means, and the maintaining of an excess pressure for a time period effected by the fourth means are repeated once or a multiple of times,
**characterized in that**
the control and regulation unit is configured for carrying out the method in accordance with claim 1.

10. A sterilization apparatus in accordance with claim 9, **characterized in that** at least one of the first and second means has a vacuum pump (50) and an aerosol container and/or aerosol generator (20).

11. A sterilization apparatus in accordance with claim 10, **characterized in that** the aerosol container and/or aerosol generator (20) is connected upstream of the sterilization chamber (10) and the vacuum pump (50) is connected downstream of the sterilization chamber (10); and **in that** the control or regulation unit controls the first means such that with open lines between the aerosol container and/or aerosol generator (20) and the sterilization chamber (10) and between the sterilization chamber (10) and the vacuum pump (50), the evacuation takes place first and then, without changing the operating mode of the sterilization apparatus, the humidifying of the sterilization chamber (10) takes place.

12. A sterilization apparatus in accordance with one of the claims 9 to 11, **characterized in that** the sterilization chamber (10) has a first connector which is in communication with a gas line for generating the excess pressure; has a second connector which is in communication with a line leading from an aerosol container; and has a third connector via which the sterilization chamber (10) is in communication with a vacuum pump (50).

13. A sterilization apparatus in accordance with claim 9, **characterized in that** the third means comprise a source for a pressurized gas; and/or **in that** the fourth means comprise one or more valves for closing the sterilization chamber (10).

## Revendications

1. Procédé de stérilisation d'un ou de plusieurs objets, comprenant les étapes :
a) placement du ou des objets dans une chambre de stérilisation (10),
b) production du vide dans la chambre de stérilisation (10) et humidification de celle-ci,
c) introduction d'un agent de stérilisation dans la chambre de stérilisation (10),
d) génération d'une surpression supérieure à la pression atmosphérique dans la chambre de stérilisation (10),
e) maintien d'une surpression dans la chambre de stérilisation (19) pendant un laps de temps,
f) répétition une ou plusieurs fois des étapes b) à e), dans lequel
l'agent de stérilisation est introduit dans la chambre de stérilisation (10) sous la forme d'un aérosol et contient de l'ozone et du peroxyde d'hydrogène et/ou de l'eau ou un ou plusieurs des produits réactionnels de ces substances, et l'aérosol se forme en introduisant de l'ozone dans un liquide, qui contient de l'eau et/ou du peroxyde d'hydrogène.

2. Procédé selon la revendication 1, **caractérisé en ce que** la surpression en particulier selon l'étape d) est d'une valeur > 1200 mbar, de préférence d'une valeur > 1500 mbar et de manière particulièrement préférentielle d'une valeur > 2000 mbar.

3. Procédé selon la revendication 1 ou 2, **caractérisé en ce que** la surpression en particulier selon l'étape e) représente une valeur de pression qui est supérieure à la pression atmosphérique pendant une partie du laps de temps ou pendant tout le laps de temps et correspond de préférence à la surpression en particulier selon l'étape d).

4. Procédé selon l'une des revendications précédentes, **caractérisé en ce que** l'humidification de la chambre de stérilisation (10) est effectuée par le fait qu'un liquide, en particulier un mélange contenant de l'eau et du peroxyde d'hydrogène, est amené à s'évaporer par l'application d'un vide, en particulier en faisant le vide et que le liquide évaporé est alimenté dans la chambre de stérilisation (10).

5. Procédé selon l'une des revendications précédentes, **caractérisé en ce que** l'introduction d'un agent de stérilisation dans la chambre de stérilisation (10) se fait à une pression dans la chambre de stérilisation qui est inférieure à la pression atmosphérique.

6. Procédé selon l'une des revendications précédentes, **caractérisé en ce que**, après l'étape f) selon la revendication 1 ou une fois la stérilisation terminée, le rinçage et dégazage de la chambre de stérilisation (10) est effectué, le rinçage et dégazage étant effectué une ou plusieurs fois.

7. Procédé selon l'une des revendications précédentes, **caractérisé en ce que** le maintien de la surpression selon l'étape e) est effectué pendant un laps de temps, dont la durée se situe dans la plage < 10 min.

8. Procédé selon l'une des revendications précédentes, **caractérisé en ce que** le laps de temps entre deux augmentations de pression successives selon l'étape d) se situe dans la plage < 20 min.

9. Dispositif de stérilisation pourvu d'une chambre de stérilisation (10), comprenant
une unité de génération de l'agent de stérilisation ainsi que d'alimentation de l'agent de stérilisation dans la chambre de stérilisation (10),
des premiers moyens destinés à produire le vide et humidifier la chambre de stérilisation (10),
des deuxièmes moyens destinés à alimenter l'agent de stérilisation dans la chambre de stérilisation (10),
des troisièmes moyens destinés à générer une surpression supérieure à la pression atmosphérique dans la chambre de stérilisation (10), ainsi que
des quatrièmes moyens destinés à maintenir une surpression dans la chambre de stérilisation (10) pour un instant ou pendant un laps de temps, dans lequel
le dispositif de stérilisation comporte une unité de commande ou de régulation, qui est conçue pour commander les premiers aux quatrièmes moyens de telle manière que la production du vide et l'humidification obtenus par les premiers moyens, l'alimentation de l'agent de stérilisation obtenue par les deuxièmes moyens, la génération d'une surpression supérieure à la pression atmosphérique obtenue par les troisièmes moyens ainsi que le maintien d'une surpression pendant un laps de temps obtenu par les quatrièmes moyens sont répétés une ou plusieurs fois,
**caractérisé en ce que**
l'unité de commande et de régulation est conçue pour l'exécution du procédé selon la revendication 1.

10. Dispositif de stérilisation selon la revendication 9, **caractérisé en ce que** au moins un des premiers et deuxièmes moyens comporte une pompe à vide (50) et un contenant à aérosol et/ou un générateur d'aérosol (20).

11. Dispositif de stérilisation selon la revendication 10, **caractérisé en ce que** le contenant à aérosol et/ou le générateur d'aérosol (20) est placé en amont de la chambre de stérilisation (10) et la pompe à vide (50) est placée en aval de la chambre de stérilisation (10) et **en ce que** l'unité de commande et de régulation commande les premiers moyens de telle manière que, lorsque les conduites entre le contenant à aérosol et/ou le générateur d'aérosol (20) et la chambre de stérilisation (10) et entre la chambre de stérilisation (10) et la pompe à vide (50) sont ouverts, d'abord la production du vide et, sans modification du mode de fonctionnement du dispositif de stérilisation, ensuite l'humidification de la chambre de stérilisation (10) sont effectuées.

12. Dispositif de stérilisation selon l'une des revendications 9 à 11, **caractérisé en ce que** la chambre de stérilisation (10) dispose d'un premier raccordement, qui est en liaison avec une conduite de gaz pour générer la surpression, elle dispose d'un deuxième raccordement, qui est en liaison avec une conduite partant d'un contenant à aérosol, et elle dispose d'un troisième raccordement, par lequel la chambre de stérilisation (10) est en liaison avec une pompe à vide (50).

13. Dispositif de stérilisation selon la revendication 9, **caractérisé en ce que** les troisièmes moyens comprennent une source pour un gaz sous pression et/ou en ce que les quatrièmes moyens comprennent une ou plusieurs vannes pour fermer la chambre de stérilisation (10).
